Europäisches Patentamt

European Patent Office

Office européen des brevets

(19)

(11) Publication number: **0 332 233 B1**

(12) **EUROPEAN PATENT SPECIFICATION**

(45) Date of publication of patent specification: **20.10.93** (51) Int. Cl.⁵: **C12N 15/52**, C12P 13/10

(21) Application number: **89108171.3**

(22) Date of filing: **16.02.84**

(60) Publication number of the earlier application in accordance with Art.76 EPC: **0 136 359**

The file contains technical information submitted after the application was filed and not included in this specification

(54) **Process for preparing L-arginine.**

(30) Priority: **17.02.83 JP 25397/83**
**17.02.83 JP 25398/83**
**28.05.83 JP 94392/83**
**29.07.83 JP 138775/83**
**04.08.83 JP 142804/83**
**24.09.83 JP 176758/83**

(43) Date of publication of application:
**13.09.89 Bulletin 89/37**

(45) Publication of the grant of the patent:
**20.10.93 Bulletin 93/42**

(84) Designated Contracting States:
**AT BE CH DE FR GB LI NL SE**

(56) References cited:
**FR-A- 2 484 448**

(73) Proprietor: **KYOWA HAKKO KOGYO CO., LTD.**
**6-1, Ohtemachi 1-chome**
**Chiyoda-ku Tokyo 100(JP)**

(72) Inventor: **Oka, Tetsuo**
**2360-17, Naramachi**

**Midori-ku**
**Yokohama-shi Kanagawa 227(JP)**
Inventor: **Katsumata, Ryoichi**
**Popuragaoka Coopo 6-401**
**12-3, Naruse 2-chome**
**Machida-shi Tokyo 194(JP)**
Inventor: **Ozaki, Akio**
**6-6, Asahimachi 3-chome**
**Machida-shi Tokyo 194(JP)**
Inventor: **Mizukami, Toru**
**14-10, Asahimachi 2-chome**
**Machida-shi Tokyo 194(JP)**
Inventor: **Yokoi, Haruhiko**
**6-6, Asahimachi 3-chome**
**Machida-shi Tokyo 194(JP)**
Inventor: **Hara, Masako**
**40-11, Sagamigaoka 5-chome**
**Zama-shi Kanagawa 228(JP)**

(74) Representative: **VOSSIUS & PARTNER**
**Postfach 86 07 67**
**D-81634 München (DE)**

Note: Within nine months from the publication of the mention of the grant of the European patent, any person may give notice to the European Patent Office of opposition to the European patent granted. Notice of opposition shall be filed in a written reasoned statement. It shall not be deemed to have been filed until the opposition fee has been paid (Art. 99(1) European patent convention).

**Description**

This invention relates to a process for producing L-arginine by a novel method for the expression of a gene. More specifically, the present invention relates to a process for producing L-arginine by transforming a host microorganism belonging to the genus Corynebacterium or Brevibacterium with a recombinant DNA of a DNA fragment containing a gene involved in the biosynthesis of L-arginine and a vector DNA, culturing the transformant in a nutrient medium, accumulating the L-arginine in the culture medium and recovering it therefrom.

For the direct production of amino acids by fermentation, methods using wild-type strains, or mutant strains such as auxotrophic strains and amino acid analog-resistant strains, of the bacteria belonging to the genus Corynebacterium, Brevibacterium, Serratia, and the like are known.

For example, the production of histidine using a strain resistant to histidine analog (Japanese Patent Publication No. 8596/81), production of tryptophan using a strain resistant to tryptophan analog (Japanese Patent Publication Nos. 4505/72 and 19037/76), production of isoleucine using a strain resistant to isoleucine analog (Japanese Patent Publication Nos. 38995/72, 6237/76 and 32070/79), production of phenylalanine using a strain resistant to phenylalanine analog (Japanese Patent Publication No. 10035/81), production of tyrosine using a strain requiring phenylalanine for its growth or being resistant to tyrosine [Agr. Chem. Soc., Japan 50 (1) R79-R87 (1976)], production of arginine using a strain resistant to L-arginine analog [Agr. Biol. Chem., 36, 1675-1684 (1972), Japanese Patent Publication Nos. 37235/79 and 150381/82] and the like are known.

The present invention relates to the production of L-arginine using a microorganism belonging to the genus Corynebacterium or Brevibacterium by recombinant DNA technology different from the conventional mutational breeding technology for the purpose of improving the L-arginine productivity.

The present inventors have constructed plasmid vectors autonomously replicable in a microorganism belonging to the genus Corynebacterium or Brevibacterium and having selectable markers and adequate cloning sites and have developed a highly efficient transformation system (Japanese Published Unexamined Patent Application Nos. 183799/82, 186492/82 and 186489/82). Further, the present inventiors have found that the plasmid vectors are useful for expressing a foreign gene in a host microorganism and increasing the productivity of amino acids by ligating a DNA fragment containing a foreign gene involved in the biosynthesis of amino acids such as glutamic acid and lysine to the plasmid vectors according to the procedures in recombinant DNA technology (Methods in Enzymology 68, Recombinant DNA, edited by Ray Wu, Academic Press 1979, U.S. Patent No. 4,237,224) and transforming Corynebacterium glutamicum L-22 or its derivatives using the transformation methods developed by the present inventors (Japanese Published Unexamined Patent Application No. 126789/83).

The present inventors have found that microorganisms prepared by the above method acquire an increased productivity of arginine.

No example of expressing a desired gene and increasing the productivity of L-arginine in a host microorganism belonging to the genus Corynebacterium or Brevibacterium by introducing a recombinant DNA containing such desired gene and vector, one of which is foreign to a host microorganism, into such host microorganism has been reported.

The present invention is explained in detail below.

The present invention provides a process for producing L-arginine by cultivating in a medium a transfornamt which is obtained by transforming a microorganism belonging to the genus Corynebacterium or Brevibacterium with a recombinant DNA of a DNA fragment containing a gene involved in the biosynthesis of L-arginine and a vector DNA.

As the DNA fragment containing the gene used in the present invention, the DNA fragment containing a gene involved in the biosynthesis of L-arginine derived from eukaryotes, prokaryotes, viruses, bacteriophages or plasmids is used. As the gene derived from prokaryotes, the gene derived from a bacterium belonging to the genus Escherichia, Corynebacterium, Brevibacterium, Microbacterium, Bacillus, Staphylococcus, Streptococcus or Serratia and involved in the biosynthesis of L-arginine or the metabolism relating to the biosynthesis is preferably used.

As the most preferable source of the gene, amino acid-producing strains and amino acid analog-resistant strains derived from the bacteria described above are used. The amino acid analog-resistance can be conferred on a plasmid after cloning.

As the enzymes involved in the biosynthesis of L-arginine, N-acetylglutamate synthetase, N-acetyl-glutamokinase, N-acetylglutamate-$\gamma$-semialdehyde dehydrogenase, N-acetylornithine-$\delta$-aminotransferase, acetylornithine deacetylase, N-acetylglutamate-acetylornithine acetyltransferase, ornithine carbamoyltransferase, arginosuccinate synthetase, argininosuccinase, and the like [Agr. Biol, Chem., 43, 1899-1903 (1979)] are mentioned.

As the gene encoding for the enzymes involved in the biosynthesis of L-arginine, the DNA carrying the genetic information of at least one of these enzymes is used.

As the DNA containing the gene involved in the biosynthesis of arginine used in the present invention, genes located convergently at around 90 minutes of the chromosomal map of Escherichia coli K-12, containing the genes encoding for acetylornithine deacetylase (argE), N-acetylglutamate-γ-semialdehyde dehydrogenase (argC), N-acetylglutamokinase (argB) and argininosuccinase (argH) [Glansdorff, N.: Genetics, 51, 167 (1965)] are mentioned.

In the example, the recombinant plasmid pEargI containing the DNA of Escherichia coli K-12 genes involved in the biosynthesis of arginine is used.

pEargI can be obtained as a recombinant of pLC20-10 and pCE53 using a host-vector system of Escherichia coli. pLC20-10 is obtained from the gene bank of Escherichia coli K-12 and is known as a plasmid carrying the genes involved in the biosynthesis of arginine described above [Clarke, L. et.: Cell, 9, 91 (1976)].

The vector used in the present invention should autonomously replicate in cells of the host microorganism. Preferably, plasmids isolated from microorganisms belonging to the genus Corynebacterium by the present inventors or derivatives thereof such as pCG1 (Japanese Published Unexamined Patent Application No. 134500/82), pCG2 Japanese Published Unexamined Patent Application No. 35197/83), pCG4 (Japanese Published Unexamined Patent Application No. 183799/82), pCE51, pCE52 (Japanese Published Unexamined Patent Application No. 126789/83), pCE53 (Japanese Published Unexamined Patent Application No. 25398/83, pCE54, pCG11, pCB100 (Japanese Published Unexamined Patent Application No. 105999/83), and the like are mentioned.

Microorganisms carrying these plasmids have been deposited with the Fermentation Research Institute, Agency of Industrial Science and Technology, and the American Type Culture Collection, U.S.A. under the following accession numbers.

| Plasmid | FERM P- | ATCC |
|---------|---------|-------|
| pCG1 | 5865 | 31808 |
| pCG2 | 5954 | 31832 |
| pCG4 | 5939 | 31830 |
| pCE54 | - | 39019 |
| pCG11 | - | 39022 |
| pCB101 | - | 39020 |

Plasmids pCE51, 52, 53 and 54 can be introduced from the plasmids mentioned above as follows.

For example, pCE51 is prepared as follows.

pCG1 is isolated from the cultured cells of Corynebacterium glutamicum 225-57 (FERM P-5865, ATCC 31808) by the method described in the specification of Japanese Published Unexamined Patent Application No. 134500/82. pGA22 is isolated from the cultured cells of Escherichia coli harboring the plasmid by a conventional method [An, G. et al.,: J. Bacteriol., 140, 400 (1979)]. Plasmid pCG1 is linearized with restriction endonuclease BgIII and a fragment of pGA22 digested with BamHI and containing kanamycin resistance (Km$^R$) gene is ligated to the linearized pCG1 using the same cohesive ends of both plasmids. Isolation of pCE51 from the ligated DNA mixture is achieved by selecting the transformants belonging to the genus Corynebacterium or Brevibacterium and containing Km$^R$ derived from pGA22, and analyzing the plasmid in the transformant.

pCE51 has a molecular weight of about 6 Kb and cleavage sites for HincII, HindIII, SmaI, XhoI and EcoRI and gives KM$^R$ phenotype.

pCE52 and pCE53 are prepared as follows.

Plasmid pCG1 is isolated from the cultured cells of Corynebacterium glutamicum 225-57 (FERM P-5865, ATCC 31808) by the method described in the above application and plasmid pGA22 is isolated from the cultured cells of Escherichia coli harboring the plasmid by a conventional method. pCG1 with a unique BgIII site is linearized with restriction enzyme BgIII and pGA22 with two BamHI sites are partially digested with BamHI. The cohesive ends of both plasmids are annealed and ligated with T4 phage DNA ligase to make a composite molecule. Selection of the recombinant plasmids from the ligation mixture is carried out by isolating transformants of the genus Corynebacterium or Brevibacterium on the basis of drug-resistances derived from pGA22 and then analyzing the plasmids in the transformants.

pCE52 and pCE53 have a molecular weight of about 10.9 Kb and cleavage sites for EcoRI, SalI, SmaI and XhoI. While pCE52 give the phenotypes of resistance to chloramphenicol ($Cm^R$) and $Km^R$, pCE53 gives resistance to tetracycline ($Tc^R$), $Cm^R$ and $Km^R$ phenotypes. Since the cleavage site for XhoI is present in the $Km^R$ gene, selection by insertional inactivation (prevention of the expression of a gene by the insertion of a DNA fragment into the gene) is possible.

Transformation with the ligated DNA mixture is carried out using protoplasts of the genus Corynebacterium or Brevibacterium, and the method described in Japanese Published Unexamined Patent Application Nos. 186492/82 and 186489/82.

Among the genes responsible for drug resistance derived from pGA22, those except for the resistance gene which is insertionally inactivated are used for selection. In no DNA adding system, transformants are recovered as a colony regenerated on a hypertonic agar medium containing generally 0.4 - 1.6 $\mu$g/m$\ell$ Tc, 2.5 - 5 $\mu$g/m$\ell$ Cm, 100 - 800 $\mu$g/m$\ell$ Km, 100 - 400 $\mu$g/m$\ell$ Sm or 200 - 1,000 $\mu$g/m$\ell$ Spec which does not allow the reversion to normal cells of the recipient protoplasts which are not treated with the ligation mixture. Alternatively, transformants are regenerated unselectively on a regeneration medium, and the resultant cells are scraped and resuspended, followed by the isolation of those cells grown on an agar medium containing a drug in a concentration wherein the recipient normal cells can not grow, that is, generally 0.5 - 4 $\mu$g/m$\ell$ Tc, 2 - 15 $\mu$g/m$\ell$ Cm, 2 - 25 $\mu$g/m$\ell$ Km, 5 - 50 $\mu$g/m$\ell$ Sm or 50 - 500 $\mu$g/m$\ell$ Spec. Some of the transformants selected by $Tc^R$, $Cm^R$ or $Km^R$ are simultaneously endowed with other drug-resistances derived from plasmid pGA22.

Plasmid DNAs in these transformants can be isolated from cultured cells of the transformants and purified according to the methods described in Japanese Published Unexamined Patent Application Nos. 134500/82 and 186489/82. The structures of the DNAs can be determined by digesting them with various restriction endonucleases and analyzing the DNA fragments by agarose gel electrophoresis. The plasmids isolated from the transformants are pCE51, pCE52 and PCE53. Recovery of plasmids from the strains is carried out according to the methods described in Japanese Published Unexamined Patent Application Nos. 134500/82, 183799/82 and 35197/83. Preparation of a recombinant DNA of a vector DNA with a DNA fragment containing a gene is carried out by conventional in vitro recombinant DNA technology.

In vitro recombinant DNA technology is carried out by cleavage and ligation of a donor DNA containing a desired gene to a vector DNA (refer to Japanese Published Unexamined Patent Application No. 126789/83, USP 4,237,224).

The ligase reaction gives recombinants containing genes other than the desired gene. The desired recombinant DNA can be obtained by directly transforming a microorganism of the genus Corynebacterium or Brevibacterium with the ligated DNA mixture, selecting the transformants having the phenotype derived from the desired gene and isolating the desired recombinant DNA from the cultured cells of the transformants. Instead of cloning the desired gene directly in a microorganism of the genus Corynebacterium or Brevibacterium, the desired gene can be cloned by using another host-vector system such as Escherichia coli. Then, it is recloned in vitro into a vector of the genus Corynebacterium or Brevibacterium to transform these microorganisms and transformants containing the desired recombinant plasmid are selected as mentioned above.

The method of cloning a gene in a host microorganism of Escherichia coli is described in, for example, Methods in Enzymology, 68, Ray Wu (ed) Academic Press New York (1979).

The following references are helpful for the construction of recombinant DNA:

S.N. Cohen, et al., U.S.P. No. 4,237,224;

Idenshi Sosa Jikkenho, edited by Yasuyuki Takagi, printed by Kodansha Scientific (1980);

Methods in Enzymology 68, Recombinant DNA, edited by Ray Wu, Academic Press, 1979

Japanese Published Unexamined Patent Application No. 126789/83.

Microorganisms belonging to the genus Corynebacterium or Brevibacterium and which are competent for incorporating DNAs may be used as the host microorganisms in the present invention. The following are examples of a suitable host microorganism.

| | Accession Number | |
| --- | --- | --- |
| | FERM P- | ATCC |
| Corynebacterium glutamicum | | 13032 |
| Corynebacterium glutamicum L-15 | 5946 | 31834 |
| Corynebacterium glutamicum LA-105 | | |
| Corynebacterium glutamicum K-38 | 7087 | |
| Corynebacterium glutamicum K-43 | 7162 | |
| Corynebacterium herculis | | 13868 |
| Corynebacterium herculis L-103 | 5947 | 31866 |
| Corynebacterium acetoacidophilum | | 13870 |
| Corynebacterium lilium | | 15990 |
| Brevibacterium divaricatum | | 14020 |
| Brevibacterium divaricatum L-204 | 5948 | 31867 |
| Brevibacterium lactofermentum | | 13869 |
| Brevibacterium lactofermentum L-312 | 5949 | 31868 |
| Brevibacterium flavum | | 14067 |
| Brevibacterium immariophilium | | 14068 |
| Brevibactrerium thiogenitalis | | 19240 |

Transformation of the host microorganisms with recombinant DNAs is carried out by the following steps:
1) Preparation of protoplasts from cultured cells;
2) Transformation of the protoplasts with a recombinant DNA;
3) Regeneration of the protoplasts to normal cells and selection of a transformant;
These steps are described in detail below.

1. Preparation of protoplasts from cultured cells:

The preparation of protoplasts is carried out by culturing a microorganisms under conditions which render it sensitive to lysozyme, a lytic enzyme, and treating the cultured cells with lysozyme in a hypertonic solution to remove the cell wall. In order to render microbial cells sensitive to lysozyme, reagents inhibiting the synthesis of bacterial cell walls are used. For example, microbial cells sensitive to lysozyme are obtained by adding, during the logarithmic growth phase, an amount of penicillin which does not inhibit or sub-inhibits the growth and then continuing culturing for several generations.

For culturing, any medium wherein the microorganism can grow may be used. For example, a nutrient medium NB (pH 7.2) consisting of 20 g/$\ell$ powdered bouillon and 5 g/$\ell$ yeast extract and a semi-synthetic medium SSM (pH 7.2) consisting of 10 g/$\ell$ glucose, 4 g/$\ell$ $NE_4Cl$, 2 g/$\ell$ urea, 1 g/$\ell$ yeast extract, 1 g/$\ell$ $KH_2PO_4$, 3 g/$\ell$ $K_2HPO_4$, 0.4 g/$\ell$ $MgCl_2 \cdot 6H_2O$, 10 mg/$\ell$ $FeSO_4 \cdot 7H_2O$, 0.2 mg/$\ell$ $MnSO_4 \cdot (4\text{-}6)H_2O$, 0.9 mg/$\ell$ $ZnSO_4 \cdot 7H_2O$, 0.4 mg/$\ell$ $CuSO_4 \cdot 5H_2O$, 0.09 mg/$\ell$ $Na_2B_4O_7 \cdot 10H_2O$, 0.04 mg/$\ell$ $(NH_4)_6MO_7O_{24} \cdot 4H_2O$, 30 $\mu$g/$\ell$ biotin and 1 mg/$\ell$ thiamine hydrochloride are used.

Microbial cells are inoculated in the medium and culturing is carried out with shaking.

The optical density (OD) of the culture medium at 660 nm is monitored with a colorimeter and penicillin, such as penicillin G, is added to the medium at an initial stage of the logarithmic growth phase (OD : 0.1 - 0.4) in a concentration of 0.1 to 2.0 U/m$\ell$. Culturing is continued to an OD value of 0.3 - 0.5, and then cells are harvested and washed with the SSM medium. the washed cells are resuspended in a suitable hypertonic medium such as PFM medium (pH 7.0 -8.5) wherein 0.4M sucrose and 0.01M $MgCl_2 \cdot 6H_2O$ are added to 2 fold diluted SSM medium and RCG medium (pH 7.0 - 8.5) consisting of 5 g/$\ell$ glucose, 5 g/$\ell$ casein hydrolysate, 2.5 g/$\ell$ yeast extract, 3.5 g/$\ell$ $K_2HPO_4$, 1.5 g/$\ell$ $KH_2PO_4$, 0.41 g/$\ell$ $MgCl_2 \cdot 6H_2O$, 10 mg/$\ell$ $FeSO_4 \cdot 7H_2O$, 2 mg/$\ell$ $MnSO_4 \cdot (4\text{-}6)H_2O$, 0.9 mg/$\ell$ $ZnSO_4 \cdot 7H_2O$, 0.4 mg/$\ell$ $CuSO_4 \cdot 5H_2O$, 0.09 mg/$\ell$ $Na_2B_4O_7 \cdot 10H_2O$, 0.04 mg/$\ell$ $(NH_4)_6Mo_7O_{24} \cdot 4H_2O$, 30 $\mu$g/$\ell$ biotin, 2 mg/$\ell$ thiamine hydrochloride and 135 g/$\ell$ sodium succinate or RCGP medium which consists of RCG medium and 3% polyvinyl pyrrolidone. To the cell suspension, lysozyme is added to a final concentration of 0.2 to 10 mg/m$\ell$, and the mixture is allowed to react at a temperature of 30 to 37°C. Protoplast formation proceeds with time and is monitored with an optical microscope. The period required for the conversion of most cells to protoplasts depends on the concentrations of the penicillin used for the lysozyme-sensitization and the amount of lysozyme used. The period is 3 - 24 hours under the conditions mentioned above.

Since protoplasts formed are destroyed under hypotonic conditions, the extent of the formation of protoplast is determined indirectly from the number of normal cells surviving under hypotonic conditions. Generally, the ratio of surviving normal cells are kept below $10^{-4}$ per lysozyme-treated normal cell.

The protoplasts as prepared above have colony-forming (regenerating) ability on a suitable hypertonic agar medium. As the agar medium, a nutrient medium, a semi-synthetic medium or a synthetic medium containing various amino acids, which contains 0.3 to 0.8M sodium succinate and 0.5 to 6% polyvinyl pyrrolidone with a molecular weight of 10,000 or 40,000 is preferably used. Generally, a semi-synthetic medium RCGP (pH 7.2) wherein 1.4% agar is added to the RCGP agar medium is used. Regeneration is carried out at a temperature of 25 to 35°C. The cultivation time required for the regeneration of protoplasts depends upon the strain used, and usually in 10 to 14 days colonies can be picked up. The efficiency of the regeneration of protoplasts on the RCGP medium also depends on the strain used, the concentrations of the penicillin added during the cultivation and the concentration of lysozyme used. The efficiency is generally $10^{-2}$ - $10^{-4}$ cells per normal cell treated with lysozyme.

2. Transformation of the protoplasts with a recombinant DNA:

Introduction of a recombinant DNA into the protoplast is carried out by mixing the protoplast and the DNA in a hypertonic solution which protects the protoplast and by adding to the mixture polyethyleneglycol (PEG, average molecular weight: 1,540 - 6,000) or polyvinylalcohol (PVA, degree of polymerization: 500 - 1,500) and a divalent metal cation which stimulates the uptake of DNA. As a stabilizing agent for the hypertonic conditions, those generally used to protect protoplasts of other microorganisms such as sucrose and sodium succinate are also employed. PEG and PVA can be used at a final concentration of 5 to 60% and 1 to 20%, respectively. Divalent metal cations such as $Ca^{++}$, $Mg^{++}$, $Mn^{++}$, $Ba^{++}$ and $Sr^{++}$ are effectively used alone or in combination at a final concentration of 1 to 100 mM. Transformation is carried out satisfactorily at 0 to 25°C.

3. Regeneration of the protoplasts to normal cells and selection of a transformant:

Regeneration of the protoplast transformed with a recombinant DNA is carried out in the same way as mentioned above by spreading the protoplasts on a hypertonic agar medium such as RCGP medium containing sodium succinate and polyvinyl pyrrolidone and incubating at a temperature wherein normal cells can grow, generally 25 to 35°C. Transformants are obtained by selecting the phenotypes derived from donor DNAs. The selection by characteristic phenotype endowed may be carried out simultaneously with regeneration on a hypertonic agar medium or may be carried out on a hypotonic agar medium after non-selective reversion to normal cells on a hypertonic agar medium.

In the case of using the lysozyme-sensitive strains described as the preferred host microorganisms, transformation may be carried out by the steps described in (1) to (3) except that the cultured cells are directly treated with lysozyme without prior treatment with penicillin in step (1). In that case, transformants are obtained at an efficiency of $10^{-2}$ to $10^{-4}$ per regenerated cell.

The following strains are the examples of the transformants obtained by the present invention.

Arginine-producing strains
Corynebacterium glutamicum K46, FERM BP-356
Corynebacterium herculis K47, FERM BP-367
Brevibacterium flavum K48, FERM BP-357

The phenotypic expression of the recombinant DNA is carried out by growing the transformant in a conventional nutrient medium. Appropriate reagents may be added to the medium according to the phenotypes expected from the gene DNA or vector DNA on the recombinant DNA.

The thus obtained transformant is cultured in a conventional manner used in the production of amino acids by fermentation. That is, the transformant is cultured in a conventional medium containing carbon sources, nitrogen sources, inorganic materials, amino acids, vitamines, etc. under aerobic conditions, with adjustment of temperature and pH. Amino acids, thus accumulated in the medium are recovered.

As the carbon source, various carbohydrates such as glucose, glycerol, fructose, sucrose, maltose, mannose, starch, starch hydrolyzate and molasses, polyalcohols and various organic acids such as pyruvic acid, fumaric acid, lactic acid and acetic acid may be used. According to the assimilability of the microorganism strain used, hydrocarbon and alcohols are employed. Blackstrap molasses is most preferably used.

As the nitrogen source, ammonia, various inorganic or organic ammonium salts such as ammonium chloride, ammonium sulfate, ammonium carbonate and ammonium acetate, urea, and nitrogenous organic substances such as peptone, NZ-amine, meat extract, yeast extract, corn steep liquor, casein hydrolyzate, fish meal or its digested product, defatted soybean or its digested product and chrysalis hydrolyzate are appropriate.

As the inorganic materials, potassium dihydrogenphosphate, dipotassium hydrogenphosphate, ammonium sulfate, ammonium chloride, magnesium sulfate, sodium chloride, ferrous sulfate, manganese sulfate, and calcium carbonate may be used. Vitamines and amine acids required for the growth of microorganisms may not be added, provided that they are supplied with other components mentioned above.

Culturing is carried out under aerobic conditions with shaking or aeration-agitation. Culturing temperature is preferably 20 to 40°C. The pH of the medium during culturing is maintained around neutral. Culturing is continued until a considerable amount of an amino acid is accumulated, generally for 1 to 5 days.

After completion of the culturing, cells are removed and the amino acid is recovered from the culture liquor by conventional manners such as treatment with active carbon or ion exchange resin.

In spite of the high similarity in microbiological characteristics, so called glutamic acid-producing microorganisms which produce glutamic acid in large amounts are classified into various species and even into different genera such as Corynebacterium and Brevibacterium, which is probably because of their industrial importance. However, it has been pointed out that these microorganisms should belong to one species because of nearly the same composition of amino acids in the cell wall and the base composition of DNAs. Recently, it has been reported that these microorganisms have at least 70 to 80% homology in DNA-DNA hybridization, indicating that these microorganisms are closely related. See, e.g., Komatsu, Y.: Report of the Fermentation Research Institute, No. 55, 1 (1980), and Suzuki, K., Kaneko, T., and Komagata, K.: Int. J. Syst. Bacteriol., 31, 131 (1981).

In consideration of the facts mentioned above, it is readily assumed that the usefulness of the present invention is applicable to all the glutamic acid-producing microorganisms. In order to stably maintain recombinant DNA molecules and express the DNA in these species, slight differences of such properties of the host microorganisms as homology in the DNA are negligible and it is sufficient for host microorganisms to allow the autonomous replication of plasmids and expression of genes on them. That these microorganisms have such abilities is apparent from the facts that plasmid pCG4 which is isolated from Corynebacterium glutamicum 225-250 (Japanese Published Unexamined Patent Application No. 183799/82) and having an streptomycin and/or spectinomycin resistant gene could replicate in the glutamic acid-producing microorganisms such as those belonging to the genus Corynebacterium or Brevibacterium and that the gene responsible for the resistance could be expressed (Japanese Published Unexamined Patent Application No. 186492/82). Further, as described in the production of histidine of the present invention, it is apparent that the gene expressed in Corynebacterium glutamicum is expressible in broad host microorganisms of the genera Corynebacterium, Brevibacterium and the like. Therefore, all the glutamic acid-producing microorganisms including those belonging to the genus Corynebacterium or Brevibacterium as well as the species Corynebacterium glutamicum are competent as the host microorganism of the present invention.

For the strains appearing in the present specification, the accession numbers, deposition date and transfer date of the deposits under the Budapest Treaty of the International Recognition of the Deposit of Microorganisms for the purposes of Patent Procedure are as follows.

| | ATCC | FERM P (BP) | Deposition date (Transfer date) Year/Month/Day |
|---|---|---|---|
| Corynebacterium glutamicum L-15 | 31334 | | 1981. 3. 9 ( ) |
| Corynebacterium herculis L-103 | 31366 | | 1981. 4. 3 ( ) |
| Brevibacterium divaricatum L-204 | 31367 | | 1981. 4. 3 ( ) |
| Brevibacterium lactofermentum L-312 | 31368 | | 1981. 4. 3 ( ) |
| Corynebacterium glutamicum LA103/pCE54 | 39019 | | 1981.12.11 |
| Corynebacterium glutamicum LA103/pCB101 | 39020 | | 1981.12.11 |
| Corynebacterium glutamicum LA103/pEthr1 | 39021 | | 1981.12.11 |
| Corynebacterium glutamicum LA103/pCG11 | 39022 | | 1981.12.11 |
| Corynebacterium glutamicum K17 | 39032 | | 1981.12.21 |
| Corynebacterium glutamicum K18 | 39033 | | 1981.12.21 |
| Corynebacterium glutamicum K19 | 39034 | | 1981.12.21 |
| Corynebacterium glutamicum K20 | 39035 | | 1981.12.21 |
| Corynebacterium glutamicum K31 | 39230 | | 1983. 1.28 |
| Corynebacterium glutamicum K32 | 39281 | | 1983. 1.28 |
| Corynebacterium herculis K33 | 39282 | | 1983. 1.28 |
| Brevibacterium flavum K34 | 39283 | | 1983. 1.28 |
| Brevibacterium lactofermentum K35 | 39284 | | 1983. 1.28 |

| | | | |
|---|---|---|---|
| Corynebacterium glutamicum K37 | 39285 | | 1983. 1.31 |
| Corynebacterium glutamicum K36 | | 6908 ( 451) | 1983. 2. 8 (1984. 1.19) |
| Corynebacterium glutamicum H33 | | 6909 ( 452) | 1983. 2. 8 (1984. 1.19) |
| Corynebacterium glutamicum C156 | | 6910 ( 453) | 1983. 2. 8 (1984. 1.19) |
| Corynebacterium glutamicum K38 | | 7087 ( 454) | 1983. 5.19 (1984. 1.19) |
| Corynebacterium glutamicum K39 | | 7088 ( 459) | 1983. 5.19 (1984. 1.21) |
| Corynebacterium glutamicum K40 | | 7160 ( 455) | 1983. 7.21 (1984. 1.19) |
| Corynebacterium glutamicum K41 | | 7161 ( 456) | 1983. 7.21 (1984. 1.19) |
| Corynebacterium glutamicum K43 | | 7162 ( 457) | 1983. 7.21 (1984. 1.19) |
| Corynebacterium glutamicum K44 | | 7163 ( 458) | 1983. 7.21 (1984. 1.19) |
| Corynebacterium glutamicum K45 | | 7164 ( 460) | 1983. 7.21 (1984. 1.21) |
| Brevibacterium fluvum K42 | | ( 355) | 1983. 9.12 |
| Corynebacterium glutamicum K46 | | ( 356) | 1983. 9.12 |
| Brevibacterium fluvum K48 | | ( 357) | 1983. 9.12 |
| Corynebacterium herculis K47 | | ( 367) | 1983. 9.21 |
| Corynebacterium glutamicum K49 | | ( 464) | 1984. 1.25 |

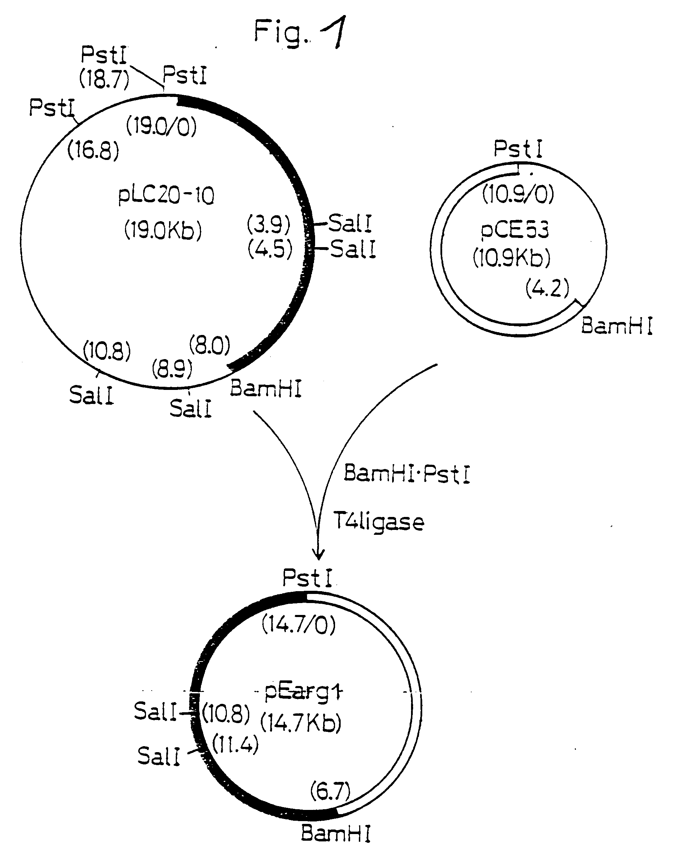

Fig. 1 illustrates the process for construction of plasmid pEargI. The restriction endonucleases used in the preparation of the cleavage map are PstI, BamHI and SalI.

Example 1

Preparation of DNA of the plasmid pCE53.

pCE53 used as a vector plasmid was isolated from Corynebacterium glutamicum L-22 harboring pCE53 as follows.

9

The strain was grown with shaking at 30°C in 400 mℓ of NB medium to an OD value of about 0.7. Cells were harvested and washed with TES buffer. The cells were suspended in 10 mℓ of the aforementioned lysozyme solution and allowed to react at 37°C for 2 hours. Then 2.4 mℓ of 5M NaCl, 0.6 mℓ of 0.5M EDTA (pH 8.5) and 4.4 mℓ of a solution consisting of 4% sodium lauryl sulfate and 0.7M NaCl were added successively. The mixture was stirred slowly and allowed to stand in an ice water bath for 15 hours.

The whole lysate was centrifuged at 4°C at 69,400 x g for 60 minutes. The supernatant fluid was recovered and 10% (by weight) polyethyleneglycol (PEG) 6,000 (product of Nakarai Kagaku Yakuhin Co.) was added. The mixture was stirred slowly to dissolve completely and then kept in an ice water bath. After 10 hours, the mixture was centrifuged at 1,500 x g for 10 minutes to recover a pellet. After the pellet was redissolved gently in 5mℓ of TES buffer, 2.0 mℓ of 1.5 mg/mℓ ethidium bromide was added. Then, cesium chloride was added calmly to adjust the density of the mixture to 1.580. The solution was centrifuged at 18°C at 105,000 x g for 48 hours. After the density gradient centrifugation, a covalently-closed circular DNA was detected under UV irradiation as a high density band located in the lower part of the centrifugation tube. The band was taken out from the side of the tube with an injector to obtain a fraction containing pCE53 DNA.

To remove ethidium bromide, the fraction was treated five times with an equal amount of cesium chloride saturated isopropyl alcohol solution consisting of 90% by volume isopropyl alcohol and 10% TES buffer solution. Then, the residue was dialysed against TES buffer solution.

pCE53 is a recombinant plasmid wherein plasmid pCG1 (Japanese Published Unexamined Patent Application No. 134500/82) of Corynebacterium glutamicum is combined with plasmid pGA22 of Escherichia coli described by An, G. et al., J. Bacteriol 140, 400 (1979). More specifically, pCE53 was constructed by inserting BglII restricted pCG1 into the BamHI site without the tetracycline-resistance gene of pGA22 and ligating by taking advantage of the same cohesive ends formed by both restriction enzymes. pCE53 has selective markers such as kanamycin-resistance derived from pGA22 and has only one cleavage site for SalI.

Example 2 Construction of microorganisms producing L-arginine:

(1) In vitro recombination of pLC20-10 and pCE54:

pLC20-10 was isolated from cultured cells of a derivative of Escherichia coli K-12 carrying the present plasmid according to the method of An [An, G. et al.: J. Bacteriol., 140, 400 (1979)]. pCE53 was isolated by the method described in Example 1.

Five units of PstI (5 units/μℓ) and 5 units of BamHI (5 units/μℓ) were added to 30 μℓ of PstI-BamHI reaction buffer solution (pH 8.0) consisting of 15 mM Tris, 10 mM $MgCl_2$, 50 mM NaCl, 25 mM $(NH_4)_2SO_4$, 1 mM mercaptoethanol and 0.01% bovine serum albumin containing 5 μg of pLC20-10 plasmid DNA. the mixture was allowed to react at 33°C for 90 minutes. Five microgram of pCE53 plasmid DNA was treated by the same method as in the treatment of pLC20-10. Both digests were heated at 65°C for 10 minutes and mixed. Then, 10μℓ of T4 ligase buffer II, 1 μℓ of 40 mM ATP, 0.3 μℓ of T4 ligase and 30 μℓ of $H_2O$ were added to the whole mixture. The reaction was carried out at 4°C for 12 hours.

(2) Recovery of pEargI:

Transformation was carried out using Escherichia coli CH754 which is a derivative of Escherichia coli K-12 and requires methionine, trytophan and arginine (defective mutation of argininosuccinase, argH). Competent cells of CH754 were prepared by the method of Dagert [Dagert, M. et al.: Gene, 6, 23 (1979)]. That is, CH754 strain was inoculated to 50 mℓ of L-broth and culturing was carried out at 37°C to an OD value at 660 nm of 0.5 by Tokyo Koden Colorimeter. The culture broth was cooled on an ice water bath for 10 minutes and centrifuged. Cells were suspended in 20 mℓ of 0.1M $CaCl_2$ cooled and allowed to stand at 0°C for 20 minutes. Cells recovered by centrifugation were suspended in 0.5 mℓ of 0.1M $CaCl_2$ and allowed to stand at 0°C for 18 hours. Fifty microliter of ligation mixture obtained above was added to 150 μℓ of the $CaCl_2$-treated cell suspension. The mixture was allowed to stand at 0°C for 10 minutes and at 37°C for 5 minutes. Then 2 mℓ of L-broth was added and culturing was carried out with shaking at 37°C for 2 hours. Cells were subjected to washing with physiological saline solution and centrifugation twice. The cells were spread on an agar medium containing 40 μg/mℓ methionine, 40 μg/mℓ trytophan and 25 μg/mℓ kanamycin. Culturing was carried out at 37°C for 3 days. A plasmid DNA was isolated from cultured cells of a developed transformant by the same method as in the isolation of pLC20-10. The plasmid DNA was digested with restriction endonucleases and analyzed by agarose gel electrophoresis. The plasmid, as illustrated in Fig. 1, has a structure wherein PstI-BamHI fragment containing the genes responsible for the biosynthesis of arginine derived from pLC20-10 and PstI-BamHI fragment containing the gene responsible for kanamycin resistance derived from pCE53 were ligated. The plasmid was named pEargI.

CH754 was retransformed using the plasmid DNA by the same method as described above. Arginine-nonrequiring transformants were obtained at a high frequency and all of them were endowed with the phenotype of kanamycin resistance. In the case that Escherichia coli CSR603 which is defective in acetylornithine deacetylase (argE) on the pathway of the biosynthesis of arginine and is a derivative of Escherichia coli K-12 was transformed, all of the transformants resistant to kanamycin were endowed with arginine-nonrequiring property.

Corynebacterium glutamicum LA291 requiring arginine for its growth was transformed by pEargI. Corynebacterium glutamicum LA291 is a mutant which is derived by a conventional mutagensis from lysozyme-sensitive mutant strain L-15 derived from Corynebacterium glutamicum ATCC 31833 and which requires arginine for its growth. It is assumed that the defective mutation depends on the loss of argininosuccinate synthetase corrsponding to argG of Escherichia coli or argininosuccinase corresponding to argH of Escherichia coli since growth of the mutant does not respond to citrulline which is a precursor two steps before arginine on the pathway of arginine biosynthesis. A seed culture of Corynebacterium glutamicum LA291 was inoculated in NB medium and culturing was carried out with shaking at 30°C. Cells were harvested at an OD value of 0.6 and suspended in an RCGP medium (pH 7.6) containing 1 mg/mℓ lysozyme at a concentration of about $10^9$ cells/mℓ. The suspension was put into an L-tube and allowed to react with gentle shaking at 30°C for 5 hours to make protoplasts.

Then, 0.5 mℓ of the protoplast suspension was transferred into a small tube and centrifuged at 2,500 x g for 5 minutes. The pellet was resuspended in 1 mℓ of TSMC buffer solution and centrifuged. The protoplasts were resuspended in 0.1 mℓ of TSMC buffer solution. Then, 100 µℓ of a mixture of a two-fold concentrated TSMC buffer solution and the pEargI plasmid DNA solution (1:1) was added to the suspension and 1.0 mℓ of TSMC buffer solution containing 20% PEG 6,000 was added. After 3 minutes, the mixture was centrifuged at 2,500 x g for 5 minutes, and the supernatant fluid was removed. The precipitated protoplasts were suspended in 1 mℓ of RCGP medium (pH 7.4), and the suspension was slowly shaken at 30°C for 2 hours. Then, 0.3 mℓ of the protoplast suspension was spread on RCGP agar medium (pH 7.4), i.e. the RCGP medium supplemented by 1.6% agar, containing 400 µg/mℓ kanamycin, and culturing was carried out at 30°C for 6 days.

All of the developed kanamycin-resistant transformants were endowed with arginine-nonrequiring property.

A plasmid DNA was isolated from cultured cells of the transformant by the same method as in the isolation of pCE53. The plasmid was digested with restriction endonucleases and analyzed by agarose gel electrophoresis to determine that the plasmid has the same structure as pEargI characterized by the cleavage pattern for various restriction endonucleases.

(3) Production of L-arginine by the pEargI-carrying strains:

The protoplasts of Corynebacterium glutamicum ATCC 13032, Corynebacterium herculis ATCC 13868 and Brevibacterium flavum ATCC 14067 were transformed with pEargI. The strains were cultured with shaking in NB medium at 30°C for 16 hours, and 0.1 mℓ of the seed culture was inoculated into 10 mℓ of SSM medium in an L-tube. Culturing was carried out at 30°C in a Monod-type culture bath, and penicillin G was added at an OD value of 0.15 to a concentration of 0.5 unit/mℓ. Culturing was continued to an OD value of about 0.6. Cells were harvested and suspended in 2 mℓ of RCGP medium (pH 7.6) containing 1 mg/mℓ lysozyme. The suspension was put in an L-tube and stirred slowly at 30°C for 14 hours to obtain protoplasts. Then, 1 mℓ of the protoplast suspension was put in a small test tube and centrifuged at 2,500 x g for 15 minutes. The protoplasts were resuspended in 1 mℓ of TSMC buffer and again subjected to centrifugation at 2,500 x g and washing. The washed protoplasts were resuspended in 0.1 mℓ of TSMC buffer solution. One hundred microliter of a mixture (1:1) of a two-fold concentrated TSMC buffer and the pEargI DNA mixtures described above was added to the protoplast suspension. Transformation was carried out using PEG 6,000 by the same method described in Example 1 for expression of the desired gene. Then, 0.3 mℓ of the mixture was spread on RCGP agar medium containing 400 µg/mℓ kanamycin and incubated at 30°C for 10 days.

Kanamycin-resistant strains were cultured with shaking in 400 mℓ of SSM medium, and penicillin G was added at an OD value of 0.15 to a concentration of 0.5 unit/mℓ. Culturing was continued to an OD value of 0.65, and cells were harvested. From the cells, plasmids were isolated by the same method as the isolation method of pCE53 in Example 1. These plasmids were digested with restriction endonucleases and analyzed by agarose gel electrophoresis. The analysis showed that the plasmids have the same structure as pEargI characterized by the cleavage pattern for various restriction endonucleases. Such transformants are Corynebacterium glutamicum K46 (FERM BP-356), Corynebacterium herculis K47 (FERM BP-367) and Brevibacterium flavum K48 (FERM BP-357.

Corynebacterium glutamicum ATCC 13032, Corynebacterium herculis ATCC 13868, Brevibacterium flavum ATCC 14067 and their pEargI-carrying strains were tested for L-arginine production as follows. The strains were cultured with shaking in NB medium at 30°C for 16 hours and 0.5 mℓ of the seed culture was inoculated in a production medium (pH 7.0) consisting of 80 g/ℓ molasses (as glucose), 4 g/ℓ $(NH_4)_2SO_4$, 0.5 g/ℓ $KH_2PO_4$, 0.5 g/ℓ $K_2HPO_4$, and 20 g/ℓ $CaCO_3$. Culturing was carried out with shaking at 30°C for 72 hours. the culture filtrate was subjected to paper chromatography and color reaction with ninhydrin. The amount of L-arginine formed was determined colorimetrically. The results are shown in Table I.

Table I

| Strain | Amount of L-arginine (mg/mℓ) |
|---|---|
| Corynebacterium glutamicum ATCC 13032 | 0 |
| Corynebacterium glutamicum K46 | 1.6 |
| Corynebacterium herculis ATCC 13868 | 0 |
| Corynebacterium herculis K47 | 1.8 |
| Brevibacterium flavum ATCC 14067 | 0 |
| Brevibacterium flavum K48 | 1.0 |

**Claims**

1.  A process for producing L-arginine which comprises culturing in a medium a microorganism obtained by transforming a host microorganism belonging to the genus Corynebacterium or Brevibacterium with a recombinant DNA wherein a DNA fragment containing the genes coding for acetylornithine deacetylase, N-acetylglutamate-$\gamma$-semialdehyde dehydrogenase, N-acetylglutamokinase and argininosuccinase which are isolated from Escherichia coli, is inserted into a vector DNA, accumulating L-arginine in the culture and recovering L-arginine therefrom.

2.  The process according to claim 1, wherein the recombinant DNA is plasmid pEargI (FERM BP-356, FERM BP-367 or FERM BP-357).

3.  The process according to claim 1, wherein the microorganism is Corynebacterium glutamicum K46, FERM BP-356, Corynebacterium herculis K47, FERM BP-367, or Brevibacterium flavum K48, FERM BP-357.

4.  A biologically pure culture of Corynebacterium glutamicum K46, FERM BP-356, Corynebacterium herculis K47, FERM BP-367, or Brevibacterium flavum K48, FERM BP-357.

**Patentansprüche**

1.  Verfahren zur Herstellung von L-Arginin, umfassend das Züchten eines Mikroorganismus, der durch Transformation eines zur Gattung Corynebacterium oder Brevibacterium gehörenden Wirtsorganismus mit einer rekombinanten DNA erhalten wurde,in einem Medium, wobei ein DNA-Fragment in eine Vektor-DNA eingefügt wird, das die Acetylornithindeacetylase, N-Acetylglutamat-$\gamma$-semialdehyddehydrogenase, N-Acetylglutamokinase und Argininsuccinase codierenden, aus Escherichia coli isolierten Gene enthält, die Anreicherung von L-Arginin in der Kultur und die Gewinnung von L-Arginin daraus.

2.  Verfahren nach Anspruch 1, wobei die rekombinante DNA das Plasmid pEargI (FERM BP-356, FERM BP-367 oder FERM BP-357) ist.

3.  Verfahren nach Anspruch 1, wobei der Mikroorganismus Corynebacterium glutamicum K46, FERM BP-356, Corynebacterium herculis K47, FERM BP-367 oder Brevibacterium flavum K48, FERM BP-357, ist.

**4.** Biologisch reine Kultur von Corynebacterium glutamicum K46, FERM BP-356, Corynebacterium herculis K47, FERM BP-367 oder Brevibacterium flavum K48, FERM BP-357.

**Revendications**

**1.** Procédé de production de la L-arginine, caractérisé en ce que l'on cultive dans un milieu un micro-organisme obtenu en transformant un micro-organisme hôte appartenant au genre Corynebacterium ou Brevibacterium avec un ADN recombinant, où un fragment d'ADN contenant les gènes codant pour l'acétylornithine désacétylase, la N-acétylglutamate-$\gamma$-semialdéhyde déshydrogénase, la N-acétylglutamokinase et l'argininosuccinase, que l'on isole d'Escherichia coli, est inséré dans un ADN vecteur, et ensuite que l'on accumule la L-arginine dans la culture et récupère la L-arginine.

**2.** Procédé suivant la revendication 1, caractérisé en ce que l'ADN recombinant est le plasmide pEargI (FERM BP-356, FERM BP-367 ou FERM BP-357).

**3.** Procédé suivant la revendication 1, caractérisé en ce que le micro-organisme est Corynebacterium glutamicum K46, FERM BP-356, Corynebacterium herculis K47, FERM BP-367 ou Brevibacterium flavum K48, FERM BP-357.

**4.** Culture biologiquement pure de Corynebacterium glutamicum K46, FERM BP-356, Corynebacterium herculis K47, FERM BP-367 ou Brevibacterium flavum K48, FERM BP-357.

# Fig. 1